# EUROPEAN PATENT SPECIFICATION

(11) **EP 2 262 510 B1**
(45) Date of publication and mention of the grant of the patent: **27.06.2012**
(21) Application number: 09710880.7
(22) Date of filing: 13.02.2009
(51) Int. Cl.: A61F 13/00, A61N 1/20, A61N 2/00, A61N 2/02, G01N 27/00, A61K 33/38

(54) **WOUND TREATMENT SYSTEM AND METHOD**
WUNDBEHANDLUNGSSYSTEM UND VERFAHREN
SYSTÈME ET PROCÉDÉ DE TRAITEMENT DES LÉSIONS

(30) Priority: 13.02.2008 GB 0802685
(43) Date of publication of application: 22.12.2010
(73) Proprietor: Pulse Medical Technologies Ltd, Suffolk IP30 9UP (GB)
(72) Inventor: HARDY, Russell, Leicestershire LE14 2UU (GB); ELDER, Iain, Suffolk IP12 1JE (GB)
(74) Representative: Humphrey-Evans, Edward John
(86) International application number: PCT/GB2009/000396
(87) International publication number: WO 2009/101411

(56) References cited:
- US-A- 5 814 094
- COMOROSAN S ET AL: "The effect of diapulse therapy on the healing of decubitus ulcer." ROMANIAN JOURNAL OF PHYSIOLOGY : PHYSIOLOGICAL SCIENCES / [ACADEMIA DE STIINTE MEDICALE] 1993 JAN-JUN, vol. 30, no. 1-2, January 1993 (1993-01), pages 41-45, XP009116708 ISSN: 1223-4974
- DUAN ET AL: "Preparation of antimicrobial poly(e-caprolactone) electrospun nanofibers containing silver-loaded zirconium phosphate nanoparticles" J. APPL. POL. SCI., vol. 106, 2007, pages 1208-1214, XP002527094
- BECKER R O: "EFFECTS OF ELECTRICALLY GENERATED SILVER IONS ON HUMAN CELLS AND WOUND HEALING" ELECTRO- AND MAGNETOBIOLOGY, NEW YORK, NY, US, vol. 19, no. 1, 1 January 2000 (2000-01-01), pages 1-19, XP001070737 ISSN: 1061-9526
- JUNGER M ET AL: "Treatment of venous leg ulcers with low frequency pulsed direct current (Dermapulse(R)): Effects on cutaneous microcirculation" HAUTARZT 1997 DE, vol. 48, no. 12, 1997, pages 897-903, XP002527481 ISSN: 0017-8470
- SHEFFET A ET AL: "Applying electric and electromagnetic energy as adjuvant treatment for pressure ulcers: a critical review." OSTOMY/WOUND MANAGEMENT FEB 2000, vol. 46, no. 2, February 2000 (2000-02), pages 28-33 , 36, XP009116709 ISSN: 0889-5899

## Description

### Field of the Invention

The present invention relates to a system and method for wound treatment which is particularly applicable to treatment of leg ulcers.

### Background to the Invention

There are 2.5 million people who suffer chronic leg ulcers in Europe which costs at least €8 billion in medical treatment Additionally, as life expectancies increase and the age profile of Europe shifts, with as many as 3% of all cases occurring in the over 65 age group, the number of age-related cases is expected to rise. Leg ulcers are slow to heal and relapse is common. In a high percentage of cases sepsis, leading to necrosis and subsequent loss of the limb is an unfortunate outcome with an additional associated human and economic cost.

However, the cost in terms of nursing resources, time lost from work and other social costs are estimated at €100 billion p.a. In terms of chronic pain, limb amputation and degradation of the quality of life for Europe's suffers the cost is many times more than this.

Various techniques for the treatment of leg ulcers have been suggested and these are discussed below:

### 1. Compression dressings

The currently accepted method of treatment involves using compression dressings which aim to improve blood flow in the limb, with frequent changing of the dressing which is in direct contact with the lesion. In addition, prolonged elevation of the limb is indicated which implies interruptions to normal work/leisure regimes. Compression reduces high venous pressure in the superficial veins and reduces oedema by reducing the pressure difference between the capillaries and the tissues. This promotes transport of metabolic products away from tissues, allowing ulcers to heal.

A variety of high compression products are available, which seem to have similar efficacy in encouraging ulcer healing. Below-knee graduated compression is the usual form of treatment to improve venous return, and to reduce venous stasis and hypertension in uncomplicated venous leg ulcers. Graduated compression delivers the highest pressure at the ankle and pressure progressively reduces towards the knee and thigh where less external pressure is needed. High compression multi-layer bandaging is recommended, usually with 3 or 4 layers. 4 layers have an improved ulcer healing rate compared to 2.

Disadvantages of compression bandaging: An appropriately trained person should apply high compression multi-layer bandaging, to avoid the risk of pressure ulceration over bony points. Active phlebitis, deep vein thrombosis, localized infection and cellulitis are all contraindications to compression.

### 2. Intermittent pneumatic compression (IPC)

IPC may improve healing rates further but good quality trials are limited. An air pump periodically inflates and deflates the device around the affected limb. A systematic review has recommended pneumatic compression devices for people with refractory oedema and significant ulceration, after 6 months of standard compression has failed, or where people are unwilling or unable to tolerate this. A Cochrane review found only small trials of IPC, which were not directly comparable. Two trials reported improved ulcer healing with IPC. There was no clear evidence that IPC improves healing when compared with compression alone or when added to standard compression regimes.

### 3. Drug treatment

A systematic review of eight randomized controlled trials suggests that Pentoxifylline which is supposed to improve blood circulation is more effective than a placebo in reducing time to complete healing, and gives additional benefit to compression. At present it is recommended only for those that have been slow to heal and it is not currently recommended for routine use. Aspirin and prostaglandins seem to show little benefit Anabolic steroids used to be recommended but are no longer mentioned. There seems very little evidence to support their use.

### 4. Hyperbaric Oxygen Therapy (HOT)

During HOT the patient goes into a closed chamber. The atmospheric pressure inside the chamber is increased. When the pressure reaches the level prescribed for the treatment, the patient is given 100 percent oxygen to breathe for a set amount of time. The patient breathes the oxygen through a hood and is advised when to take breaks and breathe the regular air inside the chamber. Some hyperbaric chambers hold only one patient but others can accommodate two or more people. On occasion, a care-giver will go into the chamber with a patient. The duration of each treatment, the number of treatments and the pressure used all vary, depending on the patient's condition. Hyperbaric oxygen therapy treatments normally take place in hospitals or private clinics. It is an extremely expensive treatment with a cost in excess of €1000 per hour per patient.

### Drawbacks to HOT:

- Pressure inside the chamber can damage the middle and inner ear.
- Some people experience claustrophobia inside the chamber.
- The therapy may affect the eyes, for example by promoting nearsightedness or cataract growth.
- A high concentration of oxygen can cause serious complications in some children who have congenital heart disease.
- As hyperbaric oxygen therapy affects blood sugar levels, diabetic should have their levels checked before and after treatment.
- Too much oxygen can sometimes, although rarely, lead to overload that can cause seizures and lung problems.
- High concentrations of oxygen at elevated pressures can pose a risk of fire.

### 5. Other treatments

• Superficial venous surgery may be considered where there is chronic venous ulceration and superficial valvular incompetence, refractory to other treatment. This may involve removal, sclerotherapy or perforating of veins.
• Sub-fascial endoscopic perforator vein surgery is still an experimental procedure.
• Skin grafting may accelerate healing if other treatments have failed.
• Low-level laser therapy is sometimes used but evidence of any benefit is poor. Combination laser and infrared light may promote healing further, but more research is needed.

### 6. Static Magneto-therapy

In 2006 the Prescription Pricing Authority of the UK's National Health Service (NHS) included static magneto-therapy in the list of treatments for chronic leg ulcers that can be prescribed on the NHS.

The currently (2007) available ambulatory magneto-therapeutic devices provide a static magnetic field generated by a permanent magnet or magnets incorporated into a band which is then secured around the leg between the knee and the calf muscle. The positioning of the band is apparently independent of the site of the ulcer and is largely governed by the shape and geometry of the limb, which provides the sole means of securing the device against the downward force of gravity.

### 7. Pulsed electro-magneto therapy (PEMT)

The most promising, although scant, peer-reviewed clinical results that have been published are in the area of magnetically augmented healing is in *pulsed* magneto-therapy applied directly over the site of the lesion. However, all of the work has been conducted using equipment which is too cumbersome to be used by the patient unaided which requires that patents make frequent visits to clinics.

In both static and stand-alone pulsed magneto-therapy the precise mechanism of how the field produces a beneficial effect remains unclear.

The electrochemical processes of the human body are extremely complex and incompletely understood. Many papers have been published on the biological effects of magnetic fields, much of it focused on the effects of radio-frequency and microwave fields or, in recent years, on fields at power-line frequencies (fifty or sixty cycles per second). Studies of the biological effects of steady magnetic fields have concentrated mostly on high fields of the level encountered in MRI magnets, typically of the order of 10,000 gauss (1 Tesla). Unfortunately, research has been very limited at field levels typical of magnetic therapy products.

The human body, like its primary constituent, water, is diamagnetic, i.e. weakly repelled by magnetic fields. In response to an applied magnetic field, the electrons in water molecules make slight adjustments in their motions, producing a net magnetic field in the opposing direction about 100,000 times smaller than the applied field. With the removal of the applied field, the electrons return to their original orbits, and the water molecules once again become nonmagnetic.

Although the diamagnetism of water and most living things is very weak, some theories suggest that magnetic fields attract blood, citing the iron it contains. However, iron in the blood is very different from metallic iron, which is strongly magnetic because the individual atomic magnets are strongly coupled together by the phenomenon of ferromagnetism. The properties of ferromagnetic materials are a result of the cooperative behaviour of many magnetic atoms acting in unison. The iron in blood consists instead of isolated iron atoms within large haemoglobin molecules, located inside the red blood cells. Although each of the iron atoms is magnetic, it is not near other iron atoms, and remains magnetically independent. The net effect of the weak paramagnetism of the isolated ion atoms in haemoglobin is only a slight decrease in the overall diamagnetism of blood. Blood, like water, is weakly repelled by magnetic fields, not attracted. More likely mechanisms for any effect, therapeutic or otherwise, are those based on magnetic forces on moving charged particles, including ions or charged molecules in flowing blood, moving across cell membranes etc.

The possible interaction mechanisms between magnetic fields and tissue that have been proposed to date may be grouped under a number of headings:
(1) Magnetite theories
(2) Free radical theories
(3) Cell membrane theories
(4) Cell nuclei theories
(5) Heat shock proteins
(6) Resonance
(7) Spatial summation
(8) Field induction
(9) Energy

While many of the above mentioned techniques are successful in their own right in treatment of leg ulcers, there remains a desire to improve treatment efficiency and costs.

Bacteria in wounds, notably chronic wounds such as ulcers, exist as both planktonic and sessile organisms. The latter are attached to a surface (e.g. biofilm form) that is postulated as a feature of chronic wounds. Bacteria behave differently in each of these two forms. This behaviour becomes relevant to bio-burden control measures when the two forms exist contemporaneously in the wound. Bacteria in planktonic form are freely accessible to topical antimicrobial agents, whereas in biofilms the bacteria are less susceptible.

The antimicrobial activity of silver has been know for many years, and numerous publications report its action against a wide variety of organisms in vitro. It is generally accepted that silver is active as Ag+ and that this species is active at low concentrations (parts per billion [ppb] or µg/L, to parts per million [ppm] or mg/L) in aqueous solutions. In a review directed at SARS (Severe Acute Respiratory Syndrome), Rentz referred to the work of von Näegeli who found Ag+ to be an active biocide at concentrations between 9.2 x 10⁻⁹ and 5.5 x 10⁻⁶ M, (i.e. 9.2 ppb and 5.5 ppm). Rentz cited a study by Cliver that reported Ag+ was active at 250 ppb in 2 hours.

The efficacy of silver ion disinfection can be illustrated by the following calculation:

At a concentration of 104 cells/mL and 50 ppb (4.7 x 10⁻⁷ mol/L) metal ions, there are approximately 2.8 x 10¹⁰ metal ions per cell.

This calculation represents a typical bacterial concentration in wound exudate and a "low" level of silver dissolution from a silver-containing dressing. However, exudate will have an influence on silver ion activity by virtue of its anion content, which bind the Ag+ ion.

Currently, little information is available that relates to the effects of silver on wound clinical isolates in the presence of common anions and protein (i.e. an exudate equivalent environment). However, Bowler et al in the document "Microbicidal properties of a silver-containing Hydrofiber dressing against a variety of burn wound pathogens", J Bum Care Rehabil. 2004; 25(2), pages 192-196, discuss a silver dressing with clinical isolates tested in a simulated wound fluid. Their findings suggest that the silver-containing dressing is likely to provide a barrier to infection. It is known that silver has the capacity to disrupt the biofilm matrix at a dose of 50 ppb.

The relationship between effective wound bed preparation and the management of wound infection through use of antimicrobial agents is acknowledged to be important in treatment The selection of any product should account for microbial sensitivity, low allergenicity, and low cellular toxicity and should not be a systemic agent.

Irrespective of the type of antimicrobial silver used in any medical device (eg, salts or metallic), the form of silver delivered to the wound should remain consistent (i.e. Ag+) and not change irrespective of the carrier dressing. However, it is generally recognized that silver efficacy is influenced by the amount of silver and its availability, which are dependent on the chosen product.

### The importance of the distribution of silver within a dressing

When the silver content and antimicrobial properties of 10 silver-containing dressings were compared in a laboratory study (published in "An in vitro analysis of the antimicrobial properties of 10 silver-containing dressings" by Thomas S, McCubbin P, J Wound Care 2003; 12(8): 305-08), highly significant differences were demonstrated in the activity of the products concerned. The researchers conclude that there are several factors that influence a dressing's ability to kill micro-organisms. One of these factors related to the distribution of the silver within the dressing (whether it is present as a surface coating or dispersed through the structure). Products that have their silver content concentrated on the surface of the dressing, instead of being bound up within their structure, performed best in these tests.

Comorsan S; et al, Romanian Journal of Physiolgy: Physiological Sciences/[Academia De Stiinte Medicale] 1993 Jan-Jun, 19930101 Vol:30, Nr:1-2, Pages:41 - 45, teaches of Diapulse Therapy, which has been around for a number of years comprises a hospital based apparatus and which is a recognised system. Duan Y-Y; et al Jouurnal of Applied Polymer Science, 20070101 J. Wiley & Sons, New York, US - ISSN 0021-8995 Vol:106, Pages:1208 - 1214 teaches of antimicrobial nanofibers of poly(E-caprolactone) (PCL) prepared by electrospinning of a PCL solution with small amounts of silver-loaded zirconium phosphate nanoparticles (nanoAgZ) for use in wound dressing applications.

A brief review of some of the silver dressings currently available clearly indicates that considerable differences exist between them in terms of their overall structure, and the concentration and formulation of the silver compound responsible for their antimicrobial activity. The products available include:
1) a two layered silver-coated, high-density polyethylene mesh, enclosing a single layer of an apertured non-woven rayon and polyester fabric. These three components are ultrasonically welded together to maintain the integrity of the dressing while in use. Silver is applied to the polyethylene mesh by a vapour deposition process which results in the formation of microscopic crystals of metallic silver. Upon activation with water, a rapid and sustained release of silver ions is provided for three or seven days. However, temporary staining of the skin can interfere with assessing the wound bed, especially when clinicians are evaluating patients who have darker pigmentation;
2) a two layered fine silver-coated mesh enclosing an inner core consisting of two layers of an apertured non-woven fabric made of rayon and polyester. Between the two layers of non-woven fabric is an additional layer of silver coated polyethylene mesh. All five layers are ultrasonically welded together to maintain the integrity of the dressing. Upon activation with water, a rapid and sustained release of silver ions is provided for three or seven days. However, temporary staining of the skin can interfere with assessing the wound bed, especially when clinicians are evaluating patients who have darker pigmentation;
3) a silver-impregnated activated charcoal cloth -this has known issues with patients who are sensitive to nylon;
4) a mixture of an alginate powder and an inorganic polymer containing ionic silver. In the presence of moisture the alginate absorbs liquid to form a gel and the silver complex breaks down in a controlled fashion, releasing ionic silver into the wound. The product is delivered either via a polyurethane film dressing or a postoperative dressing;
5) a fleece of sodium carboxymethylcellulose fibres containing 1.2% ionic silver. In the presence of exudate, the dressing absorbs liquid to form a gel, binding sodium ions and releasing silver ions;
6) a silver alginate wound dressing consisting of an absorbent foam sheet, one surface of which is coated with an alginate matrix containing ionic silver together with a cleanser, moisturizer and a superabsorbent starch co-polymer,
7) a polyurethane foam dressing that contains silver, which is released as the foam absorbs exudate;
8) a hydrocolloid dressing, which is based on established standard hydrocolloid technology but also contains a silver complex that is released by wound fluid absorbed by the dressing. This mechanism ensures a sustained release of silver ions as long as the dressing continues to absorb fluid;
9) a knitted fabric dressing that has been silver-plated by means of a proprietary autocatalytic electroless chemical (reduction-oxidation) plating technique. This technique coats the entire surface of each individual fibre from which the dressing is made, resulting in a very high silver content.
10)a synthetic, polyacrylate hydrophilic matrix in which is dispersed or suspended microscopic silver-containing particles. On exposure to moisture the silver is released into the wound in a controlled fashion;
11)a polyester mesh impregnated with carboxymethylcellulose, white soft paraffin and silver sulfadiazine (SSD);

Table 1 below illustrates experimental results of a study to identify silver concentration in the above mentioned products:

| Product | Ag content (mg/100cm⁻²) |
|---|---|
| 9 | 546 |
| 6 | 141 |
| 1,2 | 105 |
| 7 | 85 |
| 8 | 32 |
| 5 | 8.3 |
| 10 | 5.3 |
| 3 | 2.7 |

Whatever the method of silver containment used in the silver dressings known to the inventors, they all have the same constraint in that the amount of silver available at any given time at the wound bed is limited due to interaction of the Ag+ with proteins.

### Statement of Invention

According to an aspect of the present invention, there is provided a wound treatment system including a dressing which comprises a silver containment element, an exudate absorbing composition, an electromagnetic element and an encapsulation layer wherein the electromagnetic element is controllable to produce a pulsed magnetic field proximate to the silver containment element to control the number of active ions in the silver containment element.

In embodiments of the present invention, electro-magnetic enhancement of the concentration of silver ions in a wound bed is facilitated whilst minimising the total silver content required in the dressing. This will increase wound bed exposure to effective but not excessive levels of constantly replenished ionic silver over an extended period of time causing selective accumulation of silver ions within the bacterial cells and their subsequent death.

The silver-containing element may comprise a non-woven structure of polymeric nanofibres and has silver ion-loaded zirconium phosphate nanoparticles dispersed substantially uniformly in the structure.

The electromagnetic element may comprise a substantially planar spiral metal coil.

Preferably, the electromagnetic element comprises a copper coil.

The electromagnetic element may further comprise a polymeric substrate and a high-permeability magnetic layer in the form of a ceramic/polymer composite.

The dressing may further comprise an encapsulation/strike through barrier.

The exudate absorbing layer preferably has a foamed construction.

The exudate absorbing layer may include super-absorbent particles.

The system may further comprise control circuitry coupled to the electromagnetic element arranged to produce the pulsed magnetic field in the electromagnetic element on demand.

The control circuitry may be integrated with a power source for powering the electromagnetic element.

Taking into account the various aspects of existing ionic silver treatments discussed above, an advanced wound treatment system should ideally exhibit all four of the following:
1. The ability to maintain a constant optimal concentration of Ag+ ions in the wound bed.
2. A high degree of wound conformity to eliminate dead space formation.
3. An adequate fluid retention capability
4. Strike-through resistance

The present invention seeks to address the issues identified above in a pulsed, electromagnetic healing enhancement system which preferably would be incorporated into a single-use sterile wound dressing. This would accelerate ulcer healing by promoting increased concentration of ionic silver by the wound bed. This in turn will minimise the risk of sepsis, minimise cost and improve efficiency of ulcer treatment.

In particular, the present invention seeks to:
- Create a low-cost, single-use electromagnetic silver ion uptake enhancement device.
- Incorporate the electromagnetic element of the device into a silver impregnated wound dressing, whilst retaining accepted sterilization techniques.
- Produce a pocket-size electronic control unit and power supply.

Preferred embodiments of the present invention utilise a flexible electromagnetic element embedded in a disposable, sterile, silver impregnated wound dressing. The pulsed electromagnetic field would be supplied and controlled by a separate pocket-size control unit and power supply.

Embodiments of the present invention have the potential to substantially improve the treatment of ulcers and other slow-to-heal legions whilst at the same time reducing the cost of treatment. Embodiments are particularly, although not exclusively, applicable to treatment of:
- Venous and diabetic leg ulcers
- Chemical and acid burns
- Failed and relapsing skin-grafts
- Necrosis induced by ionising radiation

This in turn should:
* help reduce the number of amputations in Europe caused by slow-to-heal lesions.
* improve quality of life for 5 million people with slow-to-heal lesions across Europe each year.
* reduce the direct societal burden of health care costs.

According to another aspect of the present invention, there is provided a wound treatment method comprising: providing a wound treatment system including a silver containment element and an electromagnetic element, the electromagnet element being controllable to produce a pulsed magnetic field proximate- to the silver containment element to control the number of active ions in the silver containment element; and, controlling the electromagnet element to produce a pulsed magnetic field proximate to the silver containment element to control the number of active ions in the silver containment element.

### Brief Description of the Drawings

Embodiments of the present invention will now be described in detail, by way of example only, with reference to the accompanying drawings in which:
Figure 1 is an exploded view of a wound treatment system according to an embodiment of the present invention; and,
Figure 2 is a schematic diagram of an electromagnetic element suitable for use in the system of Figure 1.

### Detailed Description

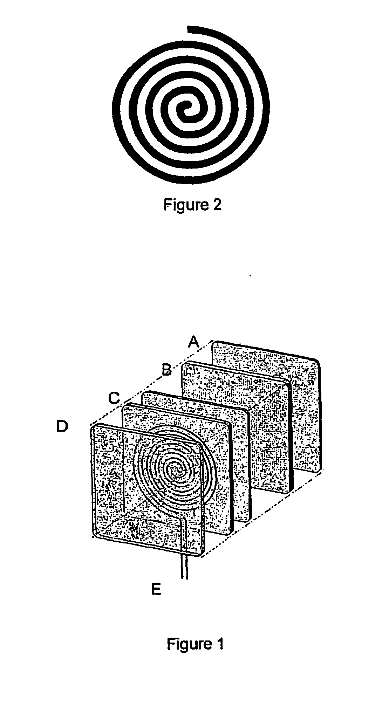

Figure 1 is an exploded view of a wound treatment system according to an embodiment of the present invention.

The system includes a silver containment layer (A), an exudate absorbing layer (B), an encapsulation layer (C), an encapsulation/strike through barrier (D) and an electromagnetic element (E).

The electromagnetic element (E), as illustrated in Figure 2, preferably comprises a planar spiral copper coil on a polymeric substrate and a high-permeability magnetic layer (equivalent to the core of a conventional electromagnet) in the form of a ceramic/polymer composite.

The copper spiral may be fabricated by Focussed Field Deposition (FFD) onto a flexible polyamide substrate. However other candidate methods of manufacture are also possible.

The ceramic/polymer composite is deposited on both sides of the substrate by stencil and screen printing, above and below the copper spiral coil.

FFD technology, which is a non-immersion additive electroplating process, was primarily designed to produce flexible circuitry using electro deposited copper extracted from copper sulphate solutions. Because copper is deposited on the substrate only where necessary and only in the amount needed, the process will significantly lower production costs. The advantages of using FFD for the copper spiral coil are that it:
- is a non-immersion process
- allows multiple substrate choice
- uses independent circuit patterns with no connections or robber bars means no waste
- has a totally controlled thicknesses capability across thickness profile
- is ecologically friendly - close circuit system - waste can be configured to go directly to drain
- can be constructed for multiple in-line metal depositions

To obtain sufficient permeability and volume magnetization for the required degree of enhancement of the magnetic field, the mass fraction of the ceramic in the ceramic/polymer composite must be greater than the mass fractions of fillers typically incorporated into polymer-matrix thick films. In general, such a high mass fraction of filler can adversely affect adhesion and can make the film susceptible to mechanical failure and delamination during flexure. These adverse effects can be overcome by:
1. Proper choice of the polymer resin and the ceramic magnetic powder filler for the film formulation, in conjunction with
2. the use of a hermetic-coating technique

Polyester resins have the best loading and adhesion characteristics. Magnetic powder comprising Manganese - Zinc ferrite particles will be used. Improved adhesion will be sought through coating with vacuum-polymerized parylene.

The silver-containing layer (A) preferably consists of a non-woven structure of polymeric nanofibres with silver ion-loaded zirconium phosphate nanoparticles, dispersed uniformly in the polymer. The nanofibres have an interwoven porous structure, high water vapour transmission ratio and high area-to-volume ratio. The high area-to-volume ratio means that the overall thickness of the dressing can be minimised in order to facilitate a high degree of dressing/wound conformity. The non-woven nature of the material means that potential liberation of dressing material upon removal from the wound site is minimised, thus reducing the risk of cross-contamination in clinical setting.

A limited degree of volume change upon exudate uptake is desirable as this will enhance the conformity of the dressing to the profile of the wound. This is addressed by the exudate absorbing layer (B) which preferably has a foamed construction.

A large volume change may give rise to folding and buckling of the foam as well as excessive pressure against the wound site, which may cause discomfort for the user. Low expanding foam may have good volume efficiency i.e. that there is low degree of unused space in the dressing when absorbing and retaining exudate.

The foam may have incorporated super-absorbent particles (SAPs). The SAPs may be incorporated into the foam in different ways, e. g. by mixing them into one or more of the components for preparation of the foam, or by impregnating or coating the foam. It is preferred that the SAPs are incorporated during the preparation of the foam, as the SAP then will be fixed in the foam and migration of SAPs into the wound is avoided. Furthermore, the SAPs will be homogeneously distributed in the foam, which may be advantageous in order to prevent blocking.

The outer strike-through barrier (D) may be of impermeable or semipermeable material. This outer material should preferably also be treated with a, say, silicone water-repellent and a fire-retardant additive. The material will be showerproof but not necessarily waterproof.

The system also includes control circuitry, a pulse generator and battery pack (not illustrated). Ideally the battery duration of the system should be at least 8 hours and preferably 10 hours. This would allow for continuous enhanced silver ion therapy for a normal day. The user would then charge the depleted battery overnight and exchange the depleted battery for a fully charged one.

The electronic control circuitry that supplies the pulsed waveform to the electromagnetic element is integral with the battery pack which is either worn on a belt, placed in a pocket or attached in some other way to the user's person.

It is important to differentiate between magneto-therapy based on modest static fields from permanent magnets, from those based on pulsed magnetic fields from electromagnets. Pulsed magnetic fields are very different from static magnetic fields because, by Maxwell's equations, time-varying magnetic fields induce electric fields.

The transient electric field associated with a pulsed magnetic field generates travelling compressions and rarefactions of ion concentrations that increase the concentration of active silver ions at any one time. For example, when the transient electric field produced by the pulsed magnetic field is at some angle into or out of the subjects skin, the silver ions in the wound bed will momentarily separate themselves into a dipole charge layer in such a way as to minimize the transient electric field at that location. When those ions are pulled toward the wound bed, they leave behind a vacancy in their concentration which is filled by adjacent ions of their own kind and in turn these ions leave a vacancy which is filled by further adjacent ions. By use of the electromagnetic element, a compression (higher than normal concentration) of ions can be achieved and maintained and this compression wave is propagated into the wound bed.

In order to make possible the ambulatory use of magneto-enhanced ionic silver therapy, embodiments of the present invention including all the control and power circuitry are made both self-contained and small enough to be worn by the patient in normal everyday conditions for extended periods (ideally up to 10 hours).

In preferred embodiments of the present invention, a 2-dimensional electromagnetic element is incorporated into a disposable silver-impregnated surgical dressing which would also allow for the use of standard compression bandaging of the limb in cases of venous leg ulcer. The embedded electromagnetic element is resistant to all standard sterilisation techniques including gamma irradiation.

The system facilitates the electro-magnetic enhancement of the concentration of silver ions in a wound bed whilst minimising the total silver content required in the dressing. This will increase wound bed exposure to effective but not excessive levels of constantly replenished ionic silver over an extended period of time causing selective accumulation of silver ions within the bacterial cells and their subsequent death.

The device could be used potentially 24 hours per day.

It is the case that as long as the device is functioning a constant concentration of silver ions will be maintained.

## Claims

1. A wound treatment system including a dressing which comprises a silver containment element (A), an exudate absorbing composition (B), an electromagnetic element (E) and an encapsulation layer (C) wherein the electromagnetic element is controllable to produce a pulsed magnetic field proximate to the silver containment element to control the number of active ions in the silver containment element.

2. A wound treatment system according to claim 1 , wherein the silver containing element comprises a non-woven structure of polymeric nanofibres and has silver ion-loaded zirconium phosphate nanoparticles dispersed substantially uniformly in the structure.

3. A wound treatment system according to claim 1 or 2, wherein the electromagnetic element comprises a substantially planar spiral metal coil.

4. A wound treatment system according to claim 3, wherein the electromagnetic element comprises a copper coil.

5. A wound treatment system according to claim 3 or 4, wherein the electromagnetic element further comprises a polymeric substrate and a high permeability magnetic layer in the form of a ceramic/polymer composite.

6. A wound treatment system according to any preceding claim, wherein the exudate absorbing composition is provided as a layer.

7. A wound treatment system according to claim 6, wherein the exudate absorbing layer has a foamed construction.

8. A wound treatment system according to claim 7, wherein the exudate absorbing layer includes super-absorbent particles.

9. A wound treatment system according to any preceding claim, further including an encapsulation/strike through barrier (D).

10. A wound treatment system according to any preceding claim, further comprising control circuitry coupled to the electromagnetic element arranged to produce the pulsed magnetic field In the electromagnetic element on demand.

11. A wound treatment system according to claim 10, wherein the control circuitry is integrated with a power source for powering the electromagnetic element.

12. A wound treatment system according to any preceding claim for use in the treatment of a wound, the steps comprising: providing a wound treatment system including a silver containment element (A), an exudate absorbing composition (B), an encapsulation layer (C) and an electromagnetic element (E), the electromagnet element being controllable to produce a pulsed magnetic field proximate to the silver containment element to control the number of active ions in the silver containment element; and, controlling the electromagnet element to produce a pulsed magnetic field proximate to the silver containment element to control the number of active ions in the silver containment element.

## Patentansprüche

1. Wundbehandlungssystem, das einen Verband beinhaltet, der eine silberhaltige Komponente (A), eine Exsudat absorbierende Zusammensetzung (B), eine elektromagnetische Komponente (E) und eine isolierende Schicht (C) aufweist, wobei die elektromagnetische Komponente gesteuert werden kann, um ein gepulstes Magnetfeld in der Nähe der silberhaltigen Komponente zu erzeugen, um die Anzahl aktiver Ionen in der silberhaltigen Komponente zu steuern.

2. Wundbehandlungssystem nach Anspruch 1, wobei die silberhaltige Komponente eine Faserverbundstruktur aus polymeren Nanofasern aufweist und silberionenbeladene Zirkoniumphosphat-Nanopartikel aufweist, die im Wesentlichen gleichförmig in der Struktur verteilt sind.

3. Wundbehandlungssystem nach Anspruch 1 oder 2, wobei die elektromagnetische Komponente eine im Wesentlichen planspiralförmige Metallwicklung aufweist.

4. Wundbehandlungssystem nach Anspruch 3, wobei die elektromagnetische Komponente eine Kupferwicklung aufweist.

5. Wundbehandlungssystem nach Anspruch 3 oder 4, wobei die elektromagnetische Komponente ferner ein polymeres Substrat und eine hochdurchlässige Magnetschicht in Form eines Keramik-Polymer-Verbundstoffs aufweist.

6. Wundbehandlungssystem nach einem der vorangehenden Ansprüche, wobei die Exsudat absorbierende Zusammensetzung als Schicht vorgesehen ist.

7. Wundbehandlungssystem nach Anspruch 6, wobei die Exsudat absorbierende Schicht einen geschäumten Aufbau aufweist.

8. Wundbehandlungssystem nach Anspruch 7, wobei die Exsudat absorbierende Schicht superabsorbierende Teilchen aufweist.

9. Wundbehandlungssystem nach einem der vorangehenden Ansprüche ferner eine Isolierung/Durchnässungssperre (D) aufweisend.

10. Wundbehandlungssystem nach einem der vorangehenden Ansprüche, ferner eine Steuerschaltung aufweisend, die mit der elektromagnetischen Komponente verkoppelt ist, die so angeordnet ist, dass sie das gepulste Magnetfeld in der elektromagnetischen Komponente auf Abruf erzeugt.

11. Wundbehandlungssystem nach Anspruch 10, wobei in die elektromagnetische Schaltung eine Leistungsquelle zur Versorgung der elektromagnetischen Komponente mit Leistung integriert ist.

12. Wundbehandlungssystem nach einem der vorangehenden Ansprüche zur Behandlung einer Wunde, die folgenden Schritte umfassend: Bereitstellen eines Wundbehandlungssystems, das ein silberhaltiges Element (A), eine Exsudat absorbierende Zusammensetzung (B), eine isolierende Schicht (C) und eine elektromagnetische Komponente (E) aufweist, wobei die elektromagnetische Komponente gesteuert werden kann, um ein gepulstes Magnetfeld in der Nähe der silberhaltigen Komponente zu erzeugen, um die Anzahl aktiver Ionen in der silberhaltigen Komponente zu steuern; und Steuern der elektromagnetischen Komponente, um ein gepulstes Magnetfeld in der Nähe der silberhaltigen Komponente zu erzeugen, um die Anzahl aktiver Ionen im silberhaltigen Komponente zu steuern.

## Revendications

1. Un système de traitement de lésions comprenant un pansement qui comprend un élément contenant de l'argent (A), une composition d'absorption d'exsudats (B), un élément électromagnétique (E) et une couche d'encapsulation (C) où l'élément électromagnétique est contrôlable de façon à produire un champ magnétique pulsé à proximité de l'élément contenant de l'argent de façon à contrôler le nombre d'ions actifs dans l'élément contenant de l'argent.

2. Un système de traitement de lésions selon la Revendication 1, où l'élément contenant de l'argent comprend une structure non tissée de nanofibres polymères et possède des nanoparticules de phosphate de zirconium chargées d'ions d'argent réparties de manière sensiblement uniforme dans la structure.

3. Un système de traitement de lésions selon la Revendication 1 ou 2, où l'élément électromagnétique comprend une bobine métallique en spirale sensiblement plane.

4. Un système de traitement de lésions selon la Revendication 3, où l'élément électromagnétique comprend une bobine en cuivre.

5. Un système de traitement de lésions selon la Revendication 3 ou 4, où l'élément électromagnétique comprend en outre un substrat polymère et une couche magnétique à haute perméabilité sous la forme d'un composé polymère/céramique.

6. Un système de traitement de lésions selon l'une quelconque des Revendications précédentes, où la composition d'absorption d'exsudats se présente sous la forme d'une couche.

7. Un système de traitement de lésions selon la Revendication 6, où la couche d'absorption d'exsudats possède une constitution en mousse.

8. Un système de traitement de lésions selon la Revendication 7, où la couche d'absorption d'exsudats contient des particules super-absorbantes.

9. Un système de traitement de lésions selon l'une quelconque des Revendications précédentes, comprenant en outre une barrière de pénétration/encapsulation (D).

10. Un système de traitement de lésions selon l'une quelconque des Revendications précédentes, comprenant en outre des circuits de commande couplés à l'élément électromagnétique et agencés de façon à produire le champ magnétique pulsé dans l'élément électromagnétique à la demande.

11. Un système de traitement de lésions selon la Revendication 10, où les circuits de commande sont intégrés à une source d'alimentation électrique destinée à alimenter l'élément électromagnétique.

12. Un système de traitement de lésions selon l'une quelconque des Revendications précédentes destiné à une utilisation dans le traitement d'une lésion comprenant les opérations suivantes : la fourniture d'un système de traitement de lésions comprenant un élément contenant de l'argent (A), une composition d'absorption d'exsudats (B), une couche d'encapsulation (C) et un élément électromagnétique (E), l'élément électromagnétique étant contrôlable de façon à produire un champ magnétique pulsé à proximité de l'élément contenant de l'argent de façon à contrôler le nombre d'ions actifs dans l'élément contenant de l'argent, et le contrôle de l'élément électromagnétique de façon à produire un champ magnétique pulsé à proximité de l'élément contenant de l'argent de façon à contrôler le nombre d'ions actifs dans l'élément contenant de l'argent.
